# EUROPEAN PATENT SPECIFICATION

(11) **EP 1 503 212 B1**
(45) Date of publication and mention of the grant of the patent: **23.05.2007**
(21) Application number: 04017838.6
(22) Date of filing: 28.07.2004
(51) Int. Cl.: G01N 33/574, A61K 39/395, C07K 14/47, C07K 14/705

(54) **Methods for diagnosis and therapy of metastasis and composition useful therein**
Verfahren zur Diagnose und Behandlung von Metastasen und dafür nützliche Zusammensetzung
Méthode de diagnostic de métastases, thérapie et composition utile à cela

(30) Priority: 28.07.2003 EP 03016586
(43) Date of publication of application: 02.02.2005
(73) Proprietor: F. HOFFMANN-LA ROCHE AG, 4070 Basel (CH)
(72) Inventor: Rio, Marie-Christine, 67400 Illkirch (FR); Tarbé De Saint Hardouin, Nesrine, 82377 Penzberg (DE); Weidle, Ulrich, 80336 München (DE)
(74) Representative: Schreiner, Siegfried

(56) References cited:
- WO-A-02/08288
- DATABASE EMBL [Online] 23 July 2003 (2003-07-23), "Rattus norvegicus mRNA for small cell adhesion glycoprotein (smagp gene)" XP002304786 retrieved from EBI accession no. EM_PRO:RNO577837 Database accession no. RNO577837
- TARBE NESRINE G ET AL: "SMAGP, a new small trans-membrane glycoprotein altered in cancer" ONCOGENE, vol. 23, no. 19, 22 April 2004 (2004-04-22), pages 3395-3403, XP002304785 ISSN: 0950-9232
- TARBE NESRINE ET AL: "Identification of rat pancreatic carcinoma genes associated with lymphogenous metastasis" ANTICANCER RESEARCH, vol. 22, no. 4, July 2002 (2002-07), pages 2015-2028, XP009039609 ISSN: 0250-7005

### SEQUENCE LISTING

<110> F. Hoffman-La Roche AG
<120> Methods for diagnosis and therapy of cancer and composition useful therein
<130> 21863
<150> EP 03016586.4
   <151> 2003-07-28
<160> 10
<170> PatentIn version 3.2
<210> 1
   <211> 291
   <212> DNA.
   <213> Homo sapiens
<220>
   <221> CDS
   <222> (1)..(291)
<400> 1
   atg aac aac cta ccg gcc aca cct tct cct gaa gaa ctg atg acc acg      48
   Met Asn Asn Leu Pro Ala Thr Pro Ser Pro Glu Glu Leu Met Thr Thr
   1               5                   10                  15
   ccg gtt ttt cag gcc cct gag aca atg tcc cca caa gct gaa gag gcc      96
   Pro Val Phe Gln Ala Pro Glu Thr Met Ser Pro Gln Ala Glu Glu Ala
               20                  25                  30
   agc aca gca ctc att gca gtt gtt ata acc gtg gtg ttc ctt acc ctg     144
   Ser Thr Ala Leu Ile Ala Val Val Ile Thr Val Val Phe Leu Thr Leu
           35                  40                  45
   ctc tca gtg gtg acc ttg atc ttc ttt tac ctg tac aag aat aaa ggc     192
   Leu Ser Val Val Thr Leu Ile Phe Phe Tyr Leu Tyr Lys Asn Lys Gly
       50                  55                  60
   agc tac gtc acc tat gag cct gca gaa ggg gag ccc agc gcc atc ctc     240
   Ser Tyr Val Thr Tyr Glu Pro Ala Glu Gly Glu Pro Ser Ala Ile Leu
   65                  70                  75                  80
   cag atg gag act gac tca gcc aag ggc aaa gag aag gaa gag tac ttc     288
   Gln Met Glu Thr Asp Ser Ala Lys Gly Lys Glu Lys Glu Glu Tyr Phe
                   85                  90                  95
   atc                                                                 291
   Ile
<210> 2
   <211> 97
   <212> PRT
   <213> Homo sapiens
<400> 2
   Met Asn Asn Leu Pro Ala Thr Pro Ser Pro Glu Glu Leu Met Thr Thr
   1 5 10 15
   Pro Val Phe Gln Ala Pro Glu Thr Met Ser Pro Gln Ala Glu Glu Ala
   20 25 30
   Ser Thr Ala Leu Ile Ala Val Val Ile Thr Val Val Phe Leu Thr Leu
   35 40 45
   Leu Ser Val Val Thr Leu Ile Phe Phe Tyr Leu Tyr Lys Asn Lys Gly
   50 55 60
   Ser Tyr Val Thr Tyr Glu Pro Ala Glu Gly Glu Pro Ser Ala Ile Leu
   65 70 75 80
   Gln Met Glu Thr Asp Ser Ala Lys Gly Lys Glu Lys Glu Glu Tyr Phe
   85 90 95
   Ile
<210> 3
   <211> 199
   <212> DNA
   <213> Artificial
<220>
   <223> primer B1
<400> 3
   ttgtggtatc cagcctcca 19
<210> 4
   <211> . 21
   <212> DNA
   <213> Artificial
<220>
   <223> primer B2
<400> 4
   ggctccaaca ctgagacact g 21
<210> 5
   <211> 20
   <212> DNA
   <213> Artificial
<220>
   <223> primer H105
<400> 5
   acaaaggcag ctacgtcacc 20
<210> 6
   <211> 20
   <212> DNA
   <213> Artificial
<220>
   <223> primer 106
<400> 6
   ctttctccat gtccctggtc 20
<210> 7
   <211> 26
   <212> DNA
   <213> Artificial
<220>
   <223> primer B140
<400> 7
   gatgaagtac tcttccttct ctttgc 26
<210> 8
   <211> 26
   <212> DNA
   <213> Artificial
<220>
   <223> primer B139
<400> 8
   aaggggagcc cagcgccatc ctccag 26
<210> 9
   <211> 20
   <212> DNA
   <213> Artificial
<220>
   <223> primer H103
<400> 9
   agaagatgga gccagcacag 20
<210> 10
   <211> 19
   <212> DNA
   <213> Artificial
<220>
   <223> primer H104
<400> 10
   atctggacga tggcactgg 19

### SEQUENCE LISTING

<110> F. Hoffman-La Roche AG
<120> Methods for diagnosis and therapy of cancer and composition useful therein
<130> 21863
<150> EP 03016586.4
   <151> 2003-07-28
<160> 10
<170> Patent In version 3.2
<210> 1
   <211> 291
   <212> DNA
   <213> Homo sapiens
<220>
   <221> CDS
   <222> (1)..(291)
<400> 1
   atg aac aac cta ccg gcc aca cct tct cct gaa gaa ctg atg acc acg        48
   Met Asn Asn Leu Pro Ala Thr Pro Ser Pro Glu Glu Leu Met Thr Thr
   1               5                   10                  15
   ccg gtt ttt cag gcc cct gag aca atg tcc cca caa gct gaa gag gcc        96
   Pro Val Phe Gln Ala Pro Glu Thr Met Ser Pro Gln Ala Glu Glu Ala
               20                  25                  30
   agc aca gca ctc att gca gtt gtt ata acc gtg gtg ttc ctt acc ctg       144
   Ser Thr Ala Leu Ile Ala Val Val Ile Thr Val Val Phe Leu Thr Leu
           35                  40                  45
   ctc tca gtg gtg acc ttg atc ttc ttt tac ctg tac aag aat aaa ggc       192
   Leu Ser Val Val Thr Leu Ile Phe Phe Tyr Leu Tyr Lys Asn Lys Gly
       50                  55                  60
   agc tac gtc acc tat gag cct gca gaa ggg gag ccc agc gcc atc ctc       240
   Ser Tyr Val Thr Tyr Glu Pro Ala Glu Gly Glu Pro Ser Ala Ile Leu
   65                  70                  75                  80
   cag atg gag act gac tca gcc aag ggc aaa gag aag gaa gag tac ttc       288
   Gln Met Glu Thr Asp Ser Ala Lys Gly Lys Glu Lys Glu Glu Tyr Phe
                   85                  90                  95
   atc                                                                   291
   Ile
<210> 2
   <211> 97
   <212> PRT
   <213> Homo sapiens
<400> 2
   Met Asn Asn Leu Pro Ala Thr Pro Ser Pro Glu Glu Leu Met Thr Thr
   1               5                   10                  15
   Pro Val Phe Gln Ala Pro Glu Thr Met Ser Pro Gln Ala Glu Glu Ala
               20                  25                  30
   Ser Thr Ala Leu Ile Ala Val Val Ile Thr Val Val Phe Leu Thr Leu
           35                  40                  45
   Leu Ser Val Val Thr Leu Ile Phe Phe Tyr Leu Tyr Lys Asn Lys Gly
       50                  55                  60
   Ser Tyr Val Thr Tyr Glu Pro Ala Glu Gly Glu Pro Ser Ala Ile Leu
   65                  70                  75                  80
   Gln Met Glu Thr Asp Ser Ala Lys Gly Lys Glu Lys Glu Glu Tyr Phe
                   85                  90                  95
   Ile
<210> 3
   <211> 19
   <212> DNA
   <213> Artificial
<220>
   <223> primer B1
<400> 3
   ttgtggtatc cagcctcca 19
<210> 4
   <211> 21
   <212> DNA
   <213> Artificial
<220>
   <223> primer B2
<400> 4
   ggctccaaca ctgagacact g 21
<210> 5
   <211> 20
   <212> DNA
   <213> Artificial
<220>
   <223> primer H105
<400> 5
   acaaaggcag ctacgtcacc 20
<210> 6
   <211> 20
   <212> DNA
   <213> Artificial
<220>
   <223> primer 106
<400> 6
   ctttctccat gtccctggtc 20
<210> 7
   <211> 26
   <212> DNA
   <213> Artificial
<220>
   <223> primer B140
<400> 7
   gatgaagtac tcttccttct ctttgc 26
<210> 8
   <211> 26
   <212> DNA
   <213> Artificial
<220>
   <223> primer B139
<400> 8
   aaggggagcc cagcgccatc ctccag 26
<210> 9
   <211> 20
   <212> DNA
   <213> Artificial
<220>
   <223> primer H103
<400> 9
   agaagatgga gccagcacag 20
<210> 10
   <211> 19
   <212> DNA
   <213> Artificial
<220>
   <223> primer H104
<400> 10
   atctggacga tggcactgg 19

## Description

The invention relates to the diagnosis of metastasis compositions therefor and therapeutic methods for the treatment of metastasis.

Cancer is the second leading cause of death in Europe and the United States, after heart disease. Cancer features uncontrolled cellular growth, which results either in local invasion of normal tissue or systemic spread of the abnormal growth. A particular type of cancer or a particular stage of cancer development may involve both elements.

The division or growth of cells in various tissues functioning in a living body normally takes place in an orderly and controlled manner. This is enabled by a delicate growth control mechanism, which involves, among other things, contact, signaling, and other communication between neighboring cells. Growth signals, stimulatory or inhibitory, are routinely exchanged between cells in a functioning tissue. Cells normally do not divide in the absence of stimulatory signals, and will cease dividing when dominated by inhibitory signals. However, such signaling or communication becomes defective or completely breaks down in cancer cells. As a result, the cells continue to divide; they invade adjacent structures, break away from the original tumor mass, and establish new growth in other parts of the body. The latter progression to malignancy is referred to as metastasis.

Cancer generally refers to malignant tumors, rather than benign tumors. Benign tumor cells are similar to normal, surrounding cells. These types of tumors are almost always encapsulated in a fibrous capsule and do not have the potential to metastasize to other parts of the body. These tumors affect local organs but do not destroy them; they usually remain small without producing symptoms for many years. Treatment becomes necessary only when the tumors grow large enough to interfere with other organs. Malignant tumors, by contrast, grow faster than benign tumors; they penetrate and destroy local tissues. Some malignant tumors may spread throughout the body via blood or the lymphatic system. The unpredictable and uncontrolled growth makes malignant cancers dangerous, and fatal in many cases. These tumors are not morphologically typical of the original tissue and are not encapsulated. Malignant tumors commonly recur after surgical removal.

Accordingly, treatment ordinarily targets malignant cancers or malignant tumors and it is exceedingly important to discover sensitive markers for early signs of cancer formation and to identify potent growth suppression agents associated therewith.

The metastatic process is a cascade of sequential steps which must all be successfully completed. The first step consists of the detachment of cancer cells from the primary tumor, followed by the degradation and invasion of the extracellular matrix (ECM) and neighbouring tissues. Once tumor cells succeeded to penetrate and survive in the lymphatic and/or the vascular system, they may arrest to a secondary site due to physical limitations (e.g. capillary size) and their ability to grow is dictated by molecular interactions of the cells with the environment in the organ (e.g. chemokines activation). Finally, recruitment of vascular supply is required to form macroscopic metastases (Chambers, A.F., et al., Nat. Rev. Cancer 2 (2002) 563-572; Welch, D.R., Clin. Exp. Metastasis 15 (1997) 272-306).

According to the great number of distinct cellular and molecular processes involved in metastasis, metastatic cells frequently display a large range of gene alterations that contribute to their metastatic potential. This includes activation of oncogenes, recruitment of metalloproteases and motility factors, as well as changes in the expression and/or functionality of multiple adhesion molecules (Hunter, K.W., et al., Cancer Res. 61 (2001) 8866-8872; Skubitz, A.P., Cancer Treat. Res. 107 (2002) 305-329). In addition, eight genes known as metastasis suppressors have been described (Steeg, P.S., Nat. Rev. Cancer 3 (2003) 55-63). Despite the steadily increasing knowledge in this field, many critical genetic and biochemical events involved in the metastatic process remain to be determined.

WO 02/08288 describes secreted and transmembrane polypeptides and nucleic acids encoding the same.

TARBE NESRINE ET AL: "Identification of rat pancreatic carcinoma genes associated with lymphogenous metastasis" ANTICANCER RESEARCH, vol. 22, no. 4, July 2002 (2002-07), pages 2015-2028, ISSN: 0250-7005 identified 210 genes which were upregulated and 247 genes which were down-regulated in a metastatic cell line.

### Summary of the Invention

It was surprisingly found that nucleic acids coding for polypeptide SMAGP (Small cell Adhesion GlycoProtein) are overexpressed in tumor cells and especially in metastatic tumor cells, whereas expression is considerably lower in normal cells Therefore, SMAGP is a valuable new target for diagnosis and therapy of cancer, preferably for colon, lung, pancreatic and breast cancer as well as for metastasis, preferably meststasis in the liver. In addition SMAGP shows a different location in early and late progression stages of tumor development and is therefore a valuable tool for differentiation of tumor stages.

The invention is defined by the appended claims. A method for determining cancer related occurence of SMAGP in a patient is provided, comprising
(i) obtaining a biological sample from a patient suspected to contain tumor cells or a part thereof;
(ii) detecting in the sample an amount of nucleic acid encoding SMAGP or an amount of polypeptide SMAGP; and
(iii) comparing the amount of said nucleic acid or polypeptide with a predetermined standard value indicating the decision line for cancer related SMAGP expression or presence in said cells and therefrom determining cancer related expression or presence of SMGAP in said patient.

A method for determining the presence or absence of cancer in a patient is provided, comprising the above described method.
A process for determining whether or not a test sample of tissue or fluid of a patient contains tumor cells or is derived from tumor cells is provided, wherein the test sample and a second sample originating from non-tumor cells from the same individual or a different individual of the same species are used, which process comprises the following steps:
   (a) incubating each respective sample under stringent hybridization conditions with a nucleic acid probe which is selected from the group consisting of:
      (i) a nucleic acid sequence of SEQ ID NO: 1, or a fragment thereof;
      (ii) a nucleic acid sequence which is complementary to any nucleic acid sequence of (i);
      (iii) a nucleic acid sequence which hybridizes under stringent conditions with the sequence of (i); and
      (iv) a nucleic acid sequence which hybridizes under stringent conditions with the sequence of (ii); and
   (b) determining the approximate amount of hybridization of each respective sample with said probe, and
   (c) comparing the approximate amount of hybridization of the test sample to an approximate amount of hybridization of said second sample to identify whether or not the test sample contains a greater amount of the specific nucleic acid or mixture of nucleic acids than does said second sample.

A method for the detection of tumor is provided, comprising
a) incubating a sample of a patient suspected of suffering from cancer, selected from the group of body fluid, of cells, or of a cell extract or cell culture supernatants of said cells, whereby said sample contains nucleic acids with a nucleic acid probe which is selected from the group consisting of
   (i) the nucleic acid shown in SEQ ID NO:1 or a nucleic acid which is complementary to said sequence, and
   (ii) nucleic acids which hybridize with one of the nucleic acids from (i) and
b) detecting hybridization, preferably by means of a further binding partner of the nucleic acid of the sample and/or the nucleic acid probe or by X-ray radiography
c) comparing the amount of said nucleic acid hybridization with a predetermined standard value indicating the decision line for cancer related SMAGP expression in said sample and therefrom determining whether said patient suffers from cancer.

Preferably said cancer is colon, lung, pancreas, or breast cancer.

Preferably, the detection is performed by the use of a binding agent which binds to SMAGP nucleic acid or polypeptide. More preferably, the binding agent is a probe hybridizing under stringent conditions with SMAGP nucleic acid or an antibody, preferably a monoclonal antibody binding to SMAGP polypeptide, preferably to the extracellular domain.

All these methods can also be performed by the use of antibodies, whereby the SMAGP polypeptide is detected by interaction between the antibody and the polypeptide.

A method for monitoring the progression of cancer is provided, especially of metastatic cancer disease in the patient. In such a method, the amount of SMAGP nucleic acid or polypeptide in a biological sample (e.g., body fluids such as blood, cell lysates or a reverse transcript of an RNA sample) of a patient suffering from cancer is determined at at least two different points in time and compared. From the change of the amount, information on the progression of said cancer can be deduced. Especially metastasis formation results in an increased occurence of SMAGP, e.g. as mRNA expression.

Diagnostic kits are provided comprising one or more oligonucleotide probes or primers for hybridization with SMAGP nucleic acid or antibody in a diagnostic assay using a sample obtained from a patient suffering from, or being suspected to have, cancer.

The invention further comprises the use of antibodies against SMAGP polypeptide in a therapeutically effective amount in the treatment of metastatic cancer. Preferably, the antibody is administered locally to the tumor of the pancreas.

The use of an antibody that binds to the polypeptide SMAGP is provided in the manufacture of a composition for inhibiting the proliferation and/or invasive potential of tumor cells. The invention further comprises the use of an antibody is provided, wherein the composition is administered to cell cultures in vitro.

The use of an antibody is provided, wherein the composition is a pharmaceutical composition and wherein the pharmaceutical composition is administered to a mammalian subject suffering from a tumor especially from a metastatic tumor

The antibody against SMAGP polypeptide is administered to the patient in a therapeutically effective amount for the treatment of cancer diseases and/or for preventing and/or inhibiting metastasis caused by cancer diseases.

### Detailed Description of the Invention

The SMAGP gene exhibits a clear association to the metastasis potential of human tumor cell lines and to tumor cells, especially from colon, breast and pancreas cancer. The encoded protein is a small transmembrane glycoprotein that is strongly conserved during evolution. Immunohistochemical analysis with specific antibodies to SMAGP indicated that this protein is localised to the lateral part of plasma membranes in normal epithelial structures. During tumor progression, SMAGP expression level and localisation are either conserved, or altered in association with disorganised epithelial structure. SMAGP is anticipated to play a role in cell-cell adhesion by interacting through its C-terminal tail with protein 4.1 and MAGUK proteins to control cytoskeleton assembly and signalling pathways.

As used herein, the term "SMAGP" means a nucleic acid encoding a polypeptide of SEQ ID NO:2, preferably the DNA sequence and the related mRNA sequence of SEQ ID NO:1 as well as the encoded polypeptide of SEQ ID NO:2. As SMAGP is a transmembrane receptor protein, the polypeptide is of outstanding interest for diagnosis and as an epitope for antibody binding the extracellular domain of SMAGP polypeptide is preferred. Therefore, it is preferred to direct the nucleic acid sample and probes to this region and especially to parts thereof which have low homology with other genes and polypeptides.

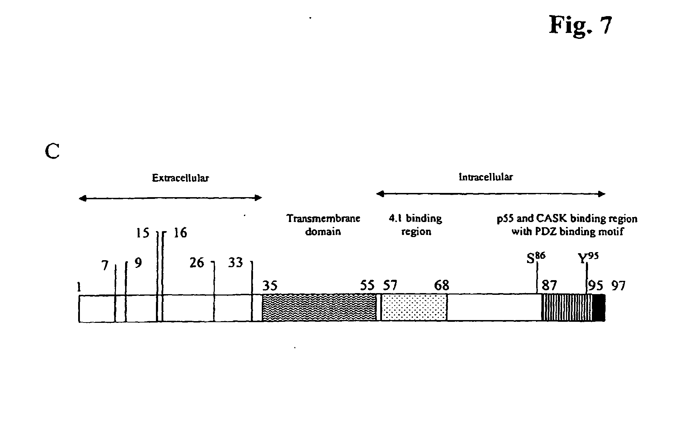

SMAGP is a transmembrane protein composed of 97 amino acids with a type III signal anchor sequence. SMAGP polypeptide contains an extracellular domain of 34 amino acids (aa 1-34), a transmembrane domain (aa 35-55) and an intracellular domain (aa 56-97), see Fig. 7.

The phrase "nucleic acid or polypeptide" as used throughout this application refers to a nucleic acid or polypeptide having a SMAGP activity which is substantially free of cellular material or culture medium when produced by recombinant DNA techniques, or substantially free of chemical precursors or other chemicals when synthesized chemically. Such a nucleic acid is preferably free of sequences which naturally flank the nucleic acid (i.e. sequences located at the 5' and the 3' ends of the nucleic acid) in the organism from which the nucleic acid is derived.

"Nucleic acid probes and primers for SMAGP" as used herein means nucleic acid fragments useful for the detection of SMAGP nucleic acids by hybridization methods. Hybridization techniques and conditions are well-known to one skilled in the art. Such hybridization conditions are, for example, moderate stringent conditions including washing with a solution of 5 x SSC, 0.5% SDS, 1.0 mmol/l EDTA, pH 8.0, followed by hybridization at 50-60°C 5 x SSC overnight, washing at room temperature for 40 minutes with 2 x SSC containing 0.1% SDS and afterwards washing with 0.1 x SSC, 0.1% SDS at 50°C for 40 min with one change of fresh solution. It is also possible to use higher temperatures for hybridization (e.g. 65-70°C) as high stringent hybridization conditions. The nucleic acid probes and primers usually consist of a SMAGP nucleic acid segment of at least about 50 contiguous positions most preferably of 200 to 300 nucleotides The optimization of the probes and primers can be performed according to the state of the art. Such probes and primers can be designed e.g. by the use of software available at (http:/lwww-genome.wi.mit.edu/genome_sohware/other/primer3.html).

For high selectivity it is preferred to use relatively low salt and/or high temperature conditions, for example, a salt concentration from about 0.02 mol/l to about 0.15 mol/l and temperatures of from about 50°C to about 70°C.

SMAGP polypeptides can be identified in diagnostic assays using specific probes and primers. Usually such methods include amplifying the target sequence in the sample by amplification methods such as the PCR method. Quantitative detection can be performed by PCR techniques, preferably by the use of quantitative RT-PCR using, e.g., the LightCycler^{®} of Roche Diagnostics GmbH, DE.

In a preferred embodiment of the invention the coding nucleic acid of the sample is amplified before the test, for example by means of the known PCR technique. Usually a derivatized (labeled) nucleic acid probe is used within the framework of nucleic acid diagnostics. This probe is contacted with a denatured DNA, RNA or RT-DNA from the sample which is bound to a carrier and in this process the temperature, ionic strength, pH and other buffer conditions are selected - depending on the length and composition of the nucleic acid probe and the resulting melting temperature of the expected hybrid - such that the labeled DNA or RNA can bind to homologous DNA or RNA (hybridization see also Wahl, G.M., et al., Proc. Natl. Acad. Sci. USA 76 (1979) 3683-3687). Suitable carriers are membranes or carrier materials based on nitrocellulose (e.g., Schleicher and Schull, BA 85, Amersham Hybond, C.), strengthened or bound nitrocellulose in powder form or nylon membranes derivatized with various functional groups (e.g., nitro groups) (e.g., Schleicher and Schüll, Nytran; NEN, Gene Screen; Amersham Hybond M.; Pall Biodyne).

To determine whether a test sample contains tumor cells, the approximate amount of hybridization of the nucleic acid with the target nucleic acid or nucleic acids is determined. The approximate amount of hybridization need not be determined quantitatively, although a quantitative determination is encompassed by the present invention. Typically, the approximate amount of hybridization is determined qualitatively, for example, by a sight inspection upon detecting hybridization. For example, if a gel is used to resolve labelled nucleic acid which hybridizes to target nucleic acid in the sample, the resulting band can be inspected visually. When performing a hybridization of isolated nucleic acid which is free from tumor cells from an individual of the same species, the same protocol is followed. One can compare the approximate amount of hybridization in the test sample to the approximate amount of hybridization in the sample free from tumor cells, to identify whether or not the test sample contains a greater amount of the target nucleic acid or nucleic acids than does the sample which is free from tumor cells.

In a further method according to the invention no second sample is used. For the detection whether the expression of SMAGP gene is upregulated, the level of mRNA of SMAGP is compared with the level of mRNA of a standard gene (housekeeping gene (see, e.g., Shaper, N.L., et al., J. Mammary Gland Biol. Neoplasia 3 (1998) 315-324; Wu, Y.Y., and Rees, J.L., Acta Derm. Venereol. 80 (2000) 2-3) of the cell, preferably by RT-PCR.

As is shown in accordance with the present invention, the SMAGP nucleic acid is expressed in a greater amount in a tumor sample than in a sample free from tumor cells. A test sample containing tumor cells will have a greater amount of the SMAGP nucleic acid than does a sample which is free from tumor cells. To identify a test sample as containing upregulated SMAGP nucleic acid, i.e., wherein the cells are tumor cells or are tumor cells of a mammary carcinoma, it is preferable that the test sample have an approximate amount of SMAGP nucleic acid which is appreciably greater that the approximate amount in a sample free of tumor cells. For example, a test sample having an upregulated SMAGP gene may have approximately 5- to approximately 60-fold greater amount of SMAGP gene than a sample free of tumor cells.

Methods of hybridization of a probe and a nucleic acid are known to a person skilled in the art and are described, for example, in WO 89/06698, EP-A 0 200 362, US 2,915,082, EP-A 0 063 879, EP-A 0 173 251, EP-A 0 128 018.

Hybridizing DNA or RNA is then detected by incubating the carrier with an antibody or antibody fragment after thorough washing and saturation to prevent unspecific binding. The antibody or the antibody fragment is directed towards the substance incorporated during hybridization to the nucleic acid probe. The antibody is in turn labeled. However, it is also possible to use a directly labeled DNA. After incubation with the antibodies it is washed again in order to only detect specifically bound antibody conjugates. The determination is then carried out according to known methods by means of the label on the antibody or the antibody fragment.

The detection of the expression can be carried out for example as:
- in situ hybridization with fixed whole cells, with fixed tissue smears,
- colony hybridization (cells) and plaque hybridization (phages and viruses),
- Southern hybridization (DNA detection),
- Northern hybridization (RNA detection),
- serum analysis (e.g., cell type analysis of cells in the serum by slot-blot analysis),
- after amplification (e.g., PCR technique).

The nucleic acids according to the invention are hence valuable markers in the diagnosis and characterization of tumors.

According to the invention inhibitors for the expression of SMAGP (e.g., antibodies or antisense nucleotides) can be used to inhibit tumor progression in vivo.

Methods for identifying and isolation of antagonists of SMAGP or inhibitors for the expression of SMAGP are provided (e.g., antibodies and antisense nucleotides). Such antagonists or inhibitors can be used to inhibit tumor progression and cause massive apoptosis of tumor cells in vivo.

Methods for identifying and isolation of SMAGP antagonists which have utility in the treatment of cancer are provided. These methods include methods for modulating the expression of the polypeptides according to the invention, methods for identifying SMAGP antagonists which can selectively bind to the proteins according to the invention, and methods of identifying SMAGP antagonists which can modulate the activity of said polypeptides. The methods further include methods for modulating, preferably inhibiting, the transcription of SMAGP gene to mRNA. These methods can be conducted in vitro or in vivo and may make use of and establish cell lines and transgenic animal models of the invention.

A SMAGP antagonist is defined as a substance or compound which decreases or inhibits the biological activity of SMAGP, a polypeptide and/or inhibits the transcription or translation of SMAGP gene. In general, screening procedures for SMAGP antagonists involve contacting candidate substances with host cells in which invasiveness is mediated by expression of SMAGP under conditions favorable for measuring SMAGP activity.

SMAGP activity may be measured in several ways. Typically, the activation is apparent by a change in cell physiology, such as increased mobility and invasiveness in vitro, or by a change in the differentiation state, or by a change in cell metabolism leading to an increase of proliferation.

The SMAGP polypeptides can be produced by recombinant means or synthetically. Non-glycosylated SMAGP polypeptide is obtained when it is produced recombinantly in prokaryotes. With the aid of the nucleic acid sequences provided by the invention it is possible to search for the SMAGP gene or its variants in genomes of any desired cells (e.g. apart from human cells, also in cells of other mammals), to identify these and to isolate the desired gene coding for the SMAGP proteins. Such processes and suitable hybridization conditions are known to a person skilled in the art and are described, for example, by Sambrook et al., Molecular Cloning: A Laboratory Manual (1989) Cold Spring Harbor Laboratory Press, New York, USA, and Hames, B.D., Higgins, S.G., Nucleic Acid Hybridisation - A Practical Approach (1985) IRL Press, Oxford, England. In this case the standard protocols described in these publications are usually used for the experiments.

With the aid of such nucleic acids coding for a SMAGP polypeptide, the polypeptide according to the invention can be obtained in a reproducible manner and in large amounts. For expression in prokaryotic or eukaryotic organisms, such as prokaryotic host cells or eukaryotic host cells, the nucleic acid is integrated into suitable expression vectors, according to methods familiar to a person skilled in the art. Such an expression vector preferably contains a regulatable/inducible promoter. These recombinant vectors are then introduced for the expression into suitable host cells such as, e.g., E. coli as a prokaryotic host cell or Saccharomyces cerevisiae, Teratocarcinoma cell line PA-1, sc 9117 (Buttner, R., et al., Mol. Cell. Biol. 11 (1991) 3573-3583), insect cells, CHO or COS cells as eukaryotic host cells and the transformed or transduced host cells are cultured under conditions which allow expression of the heterologous gene. The isolation of the protein can be carried out according to known methods from the host cell or from the culture supernatant of the host cell. Such methods are described for example by Ausubel I., Frederick M., Current Protocols in Mol. Biol. (1992), John Wiley and Sons, New York. Also in vitro reactivation of the protein may be necessary if it is not found in soluble form in the cell culture.

SMAGP polypeptide or fragments thereof can be purified after recombinant production by affinity chromatography using known protein purification techniques, including immunoprecipitation, gel filtration, ion exchange chromatography, chromatofocussing, isoelectric focussing, selective precipitation, electrophoresis, or the like and can be used for the generation of antibodies against SMAGP.

Recombinant expression vectors which are suitable for the expression of SMAGP, recombinant host cells transfected with such expression vectors, as well as a process for the recombinant production of a protein which is encoded by the SMAGP gene are provided.

Antibodies against SMAGP can be produced according to the methods known in the state of the art. For example, monoclonal or polyclonal antibodies can be produced using a polypeptide comprising the full length polypeptide or a fragment thereof. Suitable polypeptides derived from SMAGP include, for example, amino acids 83-97.

The resulting antibodies can be screened for the ability to bind to SMAGP using standard techniques such as enzyme-linked immunoabsorbent assays. Methods for identifying antigen epitopes and for the production of antibodies are described, for example, in Mole, "Epitope Mapping", In: Methods in Molecular Biology, Vol. 10, Manson (ed.), pages 105-116, The Humana Press, Inc., 1992; Price, "Production and Characterization of Synthetic Peptide-Derived Antibodies", In: Monoclonal Antibodies: Production, Engineering, and Clinical Application, Ritter and Ladyman (eds.), pp. 60-84, Cambridge University Press, 1995; Morris (ed.), Epitope Mapping Protocols 25, Humane Press, Inc., 1996; and Coligan et al. (eds.), Current Protocols in Immunology, pp. 9.3.1-9.3.5 and pp. 9.4.1-9.4.11, John Wiley & Sons, 1997.

Antibodies which are useful according to the invention, especially for therapeutic purposes, can be identified by reducing the proliferation and invasive potential of tumor cells. For this purpose, tumor cells or a tumor cell line, preferably cell line SUIT-2 007 are treated with an antibody against SMAGP and proliferation and invasive potential are measured by Cell Proliferation Reagent WST-1 (a tetrazolium salt reagent, Roche Diagnostics GmbH, DE) and Matrigel invasion assay (BDS Biosciences, www.bdbiosciences.com).

Anti-SMAGP antibodies can be derived from any animal species or are chimeric or humanized antibodies. Especially preferred are human antibodies. Human monoclonal antibodies are obtained, for example, from transgenic mice that have been engineered to produce specific human antibodies in response to antigenic challenge. In this technique, elements of the human heavy and light chain locus are introduced into strains of mice derived from embryonic stem cell lines that contain targeted disruptions of the endogenous heavy chain and light chain loci. The transgenic mice can synthesize human antibodies specific for human antigens, and the mice can be used to produce human antibody-secreting hybridomas. Methods for obtaining human antibodies from transgenic mice are described, for example, by Green, L.L., et al., Nat. Genet. 7 (1994) 13-21; Lonberg, N., et al., Nature 368 (1994) 856-859; and Taylor, L.D., et al., Int. Immun. 6 (1994) 579-591.

Monoclonal antibodies can be isolated and purified from hybridoma cultures by a variety of well-established techniques. Such isolation techniques include affinity chromatography with Protein-A Sepharose, size-exclusion chromatography, and ion-exchange chromatography (see, for example, Coligan at pages 2.7.1-2.7.12 and pages 2.9.1-2.9.3; Baines et al., "Purification of Immunoglobulin G (IgG)", In: Methods in Molecular Biology, Vol. 10, pages 79-104, The Humana Press, Inc., 1992).

The antibodies can be used for immunoassays according to the invention. Detection can be performed by contacting a biological sample with an antibody, and then contacting the biological sample with a detectably labeled molecule, which binds to the antibody. The antibody can be conjugated with avidin/streptavidin (or biotin) and the detectably labeled molecule can comprise biotin (or avidin/streptavidin). Numerous variations of this basic technique are well-known to those of skill in the art. Alternatively, an antibody can be conjugated with a detectable label to form an immunoconjugate. Suitable detectable labels include, for example, a radioisotope, a fluorescent label, a chemiluminescent label, an enzyme label, a bioluminescent label or colloidal gold. Methods of making and detecting such detectably-labeled immunoconjugates are well-known to those of ordinary skill in the art and are described in more detail below.

Preferably, the antibodies according to the invention can be used for the treatment of a patient suffering from a tumor especially from a metastatic tumor. The advantage of such a therapy with a pharmaceutical composition comprising an anti-SMAGP antibody can be demonstrated in an in vivo pancreas tumor model. Such a model is described by Alves, F., et al., Pancreas 23 (2001) 227-235. This in vivo model comprises an orthotopic transplant model for ductal adenocarcinoma in severe combined immunodeficient (SCID) mice. A suitable human colon adenocarcinoma model KM12 is described by Morikawa, K., et al., Cancer Res. 48 (1988) 6863-6871. The orthotopic transplantation of the metastatic cell lines KM12SM and KM12L4A in SCID mouse give rise to tumour and metastasis formation. A human lung tumour model based on the s.c. injection of the cell line NIH-H460 is described by Corti, C., et al., J. Cancer Res. Clin. Oncol. 122 (1996) 154-160. The cell line NIH-H460 was derived from a large-cell carcinoma of the lung and gives rise to metastases in the lungs after s.c. injection into athymic mice. A suitable breast cancer model is based on orthotopically (in the nude mouse) implanted tumours derived from breast cancer cell line MDA-MB-435 which lead to tumour and metastasis formation (John, C.M., et al., Clin. Cancer Res. 9 (2003) 2374-2383).

Generally, the dosage of administered anti-SMAGP antibodies will vary depending upon such factors as the subject's age, weight, height, sex, general medical condition and previous medical history. As an illustration, anti-SMAGP antibodies compositions can be administered at low protein doses, such as 20 to 100 milligrams 30 protein per dose, given once, or repeatedly. Alternatively, the antibodies can be administered in doses of 30 to 90 milligrams protein per dose, or 40 to 80 milligrams protein per dose, or 50 to 70 milligrams protein per dose.

Administration of antibody components to a subject can be preferably intravenous, intramuscular, by perfusion through a regional catheter, preferably directly or vicinal to the pancreas organ. The administration may be by continuous infusion or by single or multiple boluses.

A pharmaceutical composition comprising an anti-SMAGP antibody, can be formulated according to known methods to prepare pharmaceutically useful compositions, whereby the therapeutic proteins are combined in a mixture with a pharmaceutically acceptable carrier. A composition is said to be a "pharmaceutically acceptable carrier" if its administration can be tolerated by a recipient patient. Sterile phosphate-buffered saline is one example of a 0 pharmaceutically acceptable carrier. Other suitable carriers are well- known to those in the art. See, for example, Gennaro (ed.), Remington's Pharmaceutical Sciences, 19th edition, Mack Publishing Company, 1995.

For purposes of therapy, anti-SMAGP antibodies and a pharmaceutically acceptable carrier are administered to a patient in a therapeutically effective amount. A combination of the antibody and a pharmaceutically acceptable carrier is said to be administered in a "therapeutically effective amount" if the amount administered is physiologically significant.

A pharmaceutical composition comprising anti-SMAGP antibodies is preferably furnished in liquid injectable or infusable form.

The following examples, references, sequence listing and figures are provided to aid the understanding of the present invention, the true scope of which is set forth in the appended claims.

### Description of the Figures

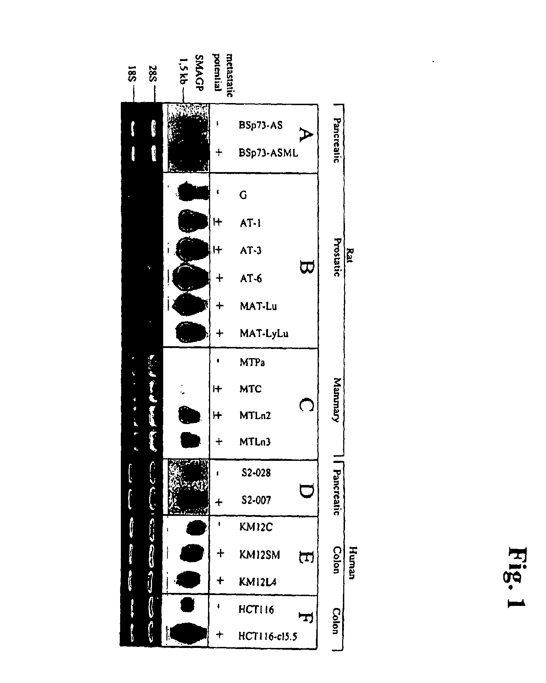

- **Fig. 1**: Northern Blot analysis for SMAGP mRNA expression in rat and human isogenic adenocarcinoma cell line systems with different metastatic potential. Agarose formaldehyde gels were loaded with 10 µg total RNA per lane. Equal loading of total RNA was assessed by ethidium bromide staining of 28S and 18S ribosomal RNAs. Non-metastatic (-), low metastatic (±) or high metastatic (+) potential is indicated for each cell line. (A) Isogenic rat pancreatic adenocarcinoma cell lines: BSp73-AS and BSp73-ASML. (B) Isogenic rat prostate adenocarcinoma cell lines: G, AT-1, AT-3, AT-6, MAT-Lu and MAT-LyLu. (C) Isogenic rat mammary adenocarcinoma cell lines: MTPa, MTC, MTLn2, and MTLn3. (D) Isogenic human pancreatic adenocarcinoma cell lines: S2-028 and S2-007. (E) Isogenic human colon adenocarcinoma cell lines: KM12C, KM12SM and KM12L4. (F) Isogenic human colon adenocarcinoma cell lines: HCT116 and HCT116-C15.5. In all systems, the highest levels of SMAGP mRNA expression were associated to cancer cells with metastatic potential.

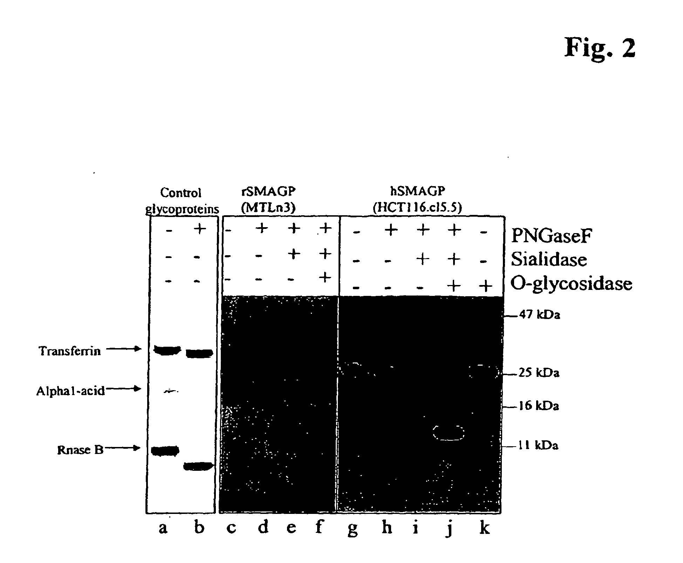

- **Fig. 2**: Immunocharacterisation of SMAGP. Western Blot analysis of SMAGP with protein extracts of MTLn3 (lanes c-f) and HCT116-c15.5 cells (lanes g-k), before and after enzymatic deglycosylation reactions with N-glycosidase F (PNGaseF), sialidase and O-glycosidase. The N-glycosylated transferrin, alpha 1-acid, and Rnase B glycoproteins were used as positive controls for N-glycosidase F treatment (lanes a-b). SMAGP is post-translationally modified by addition of sialic acid and O-glycosylation.

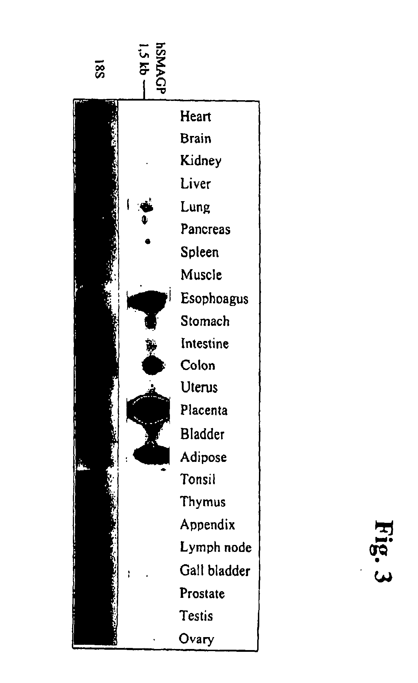

- **Fig. 3**: Northern Blot analysis for expression of SMAGP mRNA in human normal tissues. Each lane was loaded with 20 µg total RNA. Hybridization of the membrane with a 18S RNA probe was used as a loading control. Low to strong SMAGP expression is observed in almost all tissues studied.

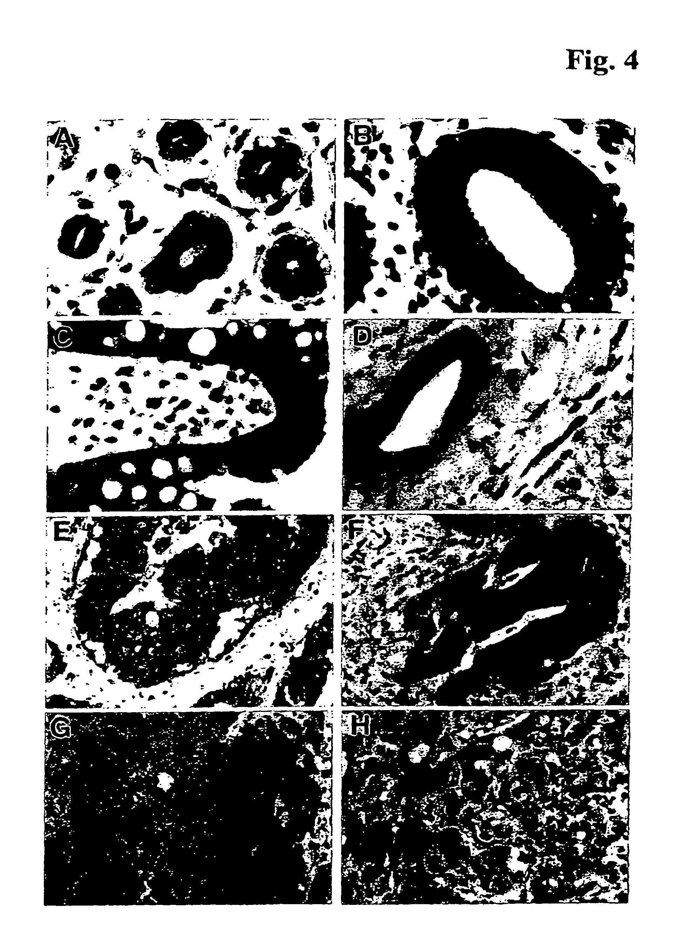

- **Fig. 4**: Immunohistochemical analysis of SMAGP expression in human normal and cancer tissues. A-D: normal breast, endometrium, colon and liver (biliary tract) tissues, respectively. SMAGP is mainly localised at the lateral sides of the plasma membranes of the epithelial cells. E-H: tumor samples from a colon cancer patient. E and G: primary tumors, F and H: liver metastases. C: normal colon tissue from the same patient. Well-differentiated tumors (E and F) exhibit strong SMAGP expression and the protein localisation to the plasma membrane is more or less conserved. By contrast, undifferentiated tumors were associated to a cytoplasmic localisation of the protein (G and H), and a decreased SMAG expression (H). Magnification: A-D, X200; E-H, X100.

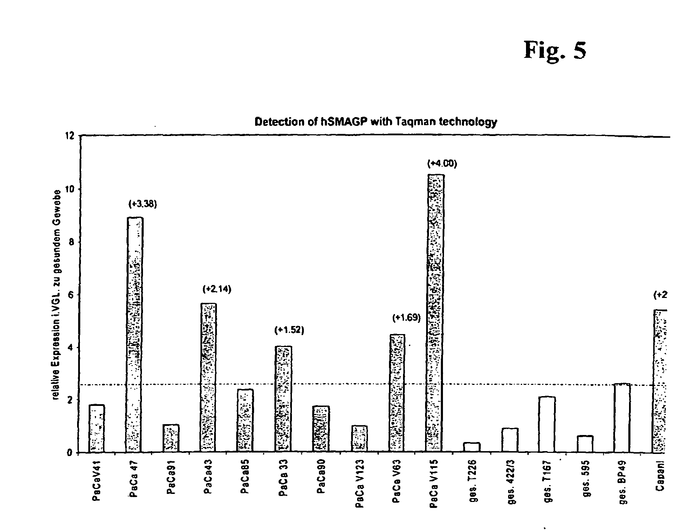

- **Fig. 5**: Detection of hSMAGP mRNA expression in human pancreas tissues.

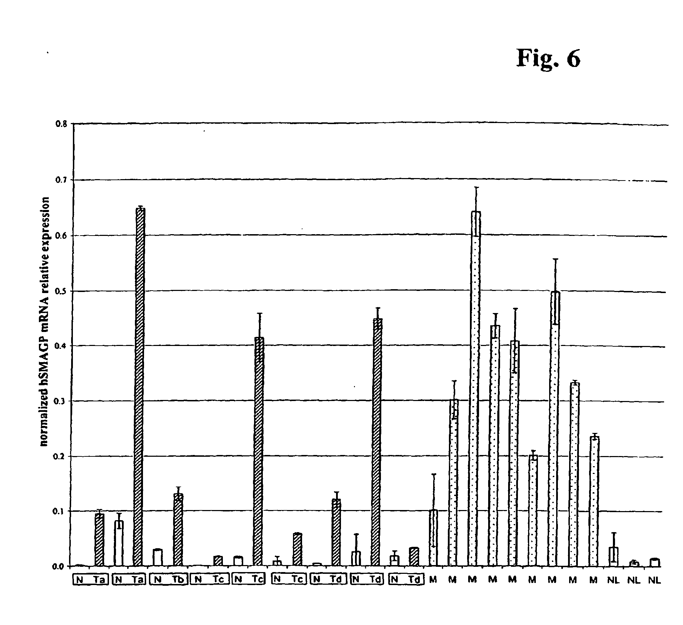

- **Fig. 6**: Expression of hSMAGP mRNA in human colon tissues measured by real-time quantitative RT-PCR. Results are expressed as normalized hSMAGP mRNA expression levels. Normal samples, primary tumours and metastases samples are indicated by white, hatched and dotted bars, respectively. Samples originated from the same patient are paired together. N, normal colon; Ta, colon tumor of Duke A stadium; Tb, colon tumor of Duke B stadium; Tc, colon tumor of Duke C stadium, Td, colon tumor of Duke D stadium; M, liver metastases; NL, normal liver.
- **Fig. 7**: Domain organization of SMAGP.

### Example 1

### Cell culture

The availability of isogenic tumor cell lines with marked differences in metastatic behaviour offers a good model to study the metastatic process. For this purpose, the rat tumor cell system BSp73 was used, comprising two stable variants derived by serial transplantation of a spontaneous rat pancreatic tumor: the non-metastatic BSp73-AS variant and the metastatic BSp73-ASML variant (Matzku, S., et al.,Invasion Metastasis 3 (1983) 109-123). Previous studies have demonstrated that it constitutes an appropriate cellular model to identify metastasis-associated genes. Monoclonal antibodies were raised against membrane proteins of the metastatic BSp73-ASML cell line in order to identify surface molecules associated with the metastatic phenotype and four proteins (CD44v, D6.1A, C4.4A and EGP314) only present on the surface of the metastastic variant were identified. Their involvement in the metastasis process was demonstrated using stable transfectants of several non-metastasising tumor cells. Gene expression was sufficient to either confer metastatic potential or to promote distinct steps of the metastatic cascade (Claas, C., et al., J. Cell. Biol. 141 (1998) 267-280; Gunthert, U., et al., Cell 65 (1991) 13-24; Rosel, M., et al., Oncogene 17 (1998) 1989-2002; and Wurfel, J., et al., Oncogene 18 (1999) 2323-2334). Furthermore, the deregulated expression of CD44v has been correlated to a poor prognosis in many human cancers

Rat pancreatic adenocarcinoma cell lines BSp73-AS and BSp73-ASML are described by Matzku, S., et al.,Invasion Metastasis 3 (1983) 109-123. Rat prostatic adenocarcinoma cell lines G, AT-1, AT-3, AT-6, MAT-Lu, and MAT-LyLu (Isaacs, J.T., et al., Prostate 9 (1986) 261-281 were purchased at the European Collection of Cell Cultures (ECACC, Salisbury, UK). Human colon adenocarcinoma cell lines KM12C, KM12SM, and KM12L4 are described by Morikawa, K., et al., Cancer Res. 48 (1988) 6863-6871. Human colon adenocarcinoma cell lines HCT116 is described by Brattain, M.G., et al., Cancer Res. 41 (1981) 1751-1756. HCT116-cl5.5 was isolated *in vivo* from HCT116 derived lung metastases and was identified as highly metastatic. The above mentioned cell lines were cultured without antibiotics in RPMI 1640 supplemented with 10% foetal bovine serum and 2mM L-glutamine. Rat mammary adenocarcinoma cell lines MTPa, MTC, MTLn2 and MTLn3 are described by Neri, A., et al., J. Natl. Cancer Inst. 68 (1982) 507-517 and were cultured in MEM-α supplemented with 10% foetal bovine serum. Human pancreatic adenocarcinoma cell lines S2-028 and S2-007 are described by Taniguchi, S., et al., Clin. Exp. Metastasis 10 (1992) 259-266 and were cultured in D-MEM supplemented with 10% foetal bovine serum and 2mM L-glutamine. All cell lines were free of mycoplasma contamination.

### Example 2

### Northern Blotting

Total RNA was isolated from frozen cell pellets with RNeasy midi kit (Qiagen, Hilden, Germany). Ten µg of total RNA were size-separated on a denaturing 1% agarose formaldehyde gel and blotted (NorthernMax^{™} blotting kits, Ambion) onto nylon membrane (BrightStar-Plus^{™}, Ambion, Austin, USA). After UV-crosslinking (UV Stratalinker 2400, Stratagene, La Jolla, USA), blots were hybridised with [α-³²P]dATP-labeled cDNA (Tarbé, N., et al., Anticancer Res. 22 (2002) 2015-2027). Equal loading of total RNA on agarose-formaldehyde gel was verified by ethidium bromide staining of ribosomal RNA. Expression of SMAGP mRNA in human normal tissues was determined by use of commercial human total RNA blots (Northern Territory^{™}, Invitrogen, Huntsville, USA). Each lane was loaded with 20 µg total RNA and equal loading was checked by quantification of 18S RNA. Blots were processed as mentioned above. cDNA probes for the rat and human mRNA of SMAGP were realised with RT-PCR, using the primer pairs
B1 (TTGTGGTATCGAGCCTCCA) (SEQ ID NO:3) /
B2 (GGCTCCAACACTGAGACACTG) (SEQ ID NO:4) and
H105 (ACAAAGGCAGCTACGTCACC) (SEQ ID NO:5)
106 (CTTTCTCCATGTCCCTGGTC) (SEQ ID NO:6), respectively.

The resulting probes correspond to a 290 bp and 295 bp region in the 3'UTR of rat and human SMAGP mRNA, respectively.

### Example 3

### RACE-PCR

Full length rSMAGP cDNA sequence was obtained with RACE-PCR using a SMART^{™} RACE cDNA Amplification kit (Clontech, Palo Alto, USA). 1 µg total RNA from BSp73-ASML cell line was reverse transcripted according to manufacturer's instructions. The 5' and 3' untranslated regions were amplified by PCR with Clontech Universal primers and rSMAGP sequence specific primers B140 (5'-GAT GAA GTA CTC TTC CTT CTC TTT GC- 3') (SEQ ID NO:7) and B139 (5'-AAG GGG AGC CCA GCG CCA TCC TCC AG-3') (SEQ ID NO:8), respectively. The PCR products were cloned into pCR^{®} 4-TOPO vector (Invitrogen) and sequenced with ABI PRISM® 310 Genetic Analyzer (Applied Biosystems, Foster City, USA).

### Example 4

### Antibodies and Western Blotting

Rabbit polyclonal antibody anti-SMAGP was generated by Eurogentech S.A. (Herstal, Belgium). The synthetic peptide ESDLAKGSEKEEYFI, corresponding to residues 83-97 of hSMAGP (SEQ ID NO:2), was coupled to KLH (keyhole limpet hemocyanin) and injected into rabbits. Immunoreactive sera against the synthetic peptide were affinity-purified. Monoclonal rabbit antibodies can be generated according to Spieker-Polet, H., et al., Proc. Natl. Acad. Sci. USA 92 (1995) 9348-9352.

For Western Blotting, proteins were extracted with RIPA buffer (50mM Tris-Cl pH 7.5, 150mM NaCl, 1% NP-40, 0.5% sodium deoxycholate, 0.1% SDS) containing a protease inhibitor mixture (Complete EDTA free protease Inhibitors, Roche Diagnostics, Mannheim, Germany). Polyacrylamide gel electrophoresis was performed under reducing conditions using NuPAGE® 12% Bis-Tris gel (Invitrogen, Carlsbad, USA) and NuPAGE® MES SDS running buffer (Invitrogen). Gels were semi-dry blotted to a nitrocellulose membrane and blocked in TBS buffer (100 mM Tris-Cl pH7.5, 150mM NaCl) plus 5% non-fat dry milk (Merck, Darmstadt, Germany). After incubation with rabbit anti-SMAGP diluted 1:5000 as primary antibody, membranes were washed in TBS buffer with 0.1% Tween20, incubated with horseradish peroxidase-conjugated anti-rabbit (Roche Diagnostics) as secondary antibody and washed again. Antibody detection was performed using enhanced chemiluminescence (Lumi-light^{PLUS} Western Blotting substrate, Roche Diagnostics GmbH, Germany).

### Example 5

### Deglycosylation assay

Enzymatic deglycosylation experiments were performed with 100 µg or less of total cell lysat denatured at 100°C for 5 min in reaction buffer (0.05mM sodium phosphate, pH7) and denaturation buffer (2% SDS, 1M beta-mercaptoethanol). Triton X-100 was then added to the solution to a final concentration of 10%. The enzymes N-glycosidaseF (PNGaseF), Sialidase, and O-glycosidase (Sigma, Steinheim, Germany) were added to a final concentration of 100 U/ml, 100 mU/ml and 25 mU/ml, respectively, in a total volume of 50 µl. The samples were incubated for 3h at 37°C. Human transferrin, alphal-acid and RNaseB glycoproteins were used as positive controls for PNGaseF activity, analysed on SDS-PAGE under reducing conditions, and visualised by coomassie blue staining. After deglycosylation reactions SMAGP was analysed by Western Blotting with rabbit anti-SMAGP antibody.

### Example 6

### Immunohistochemistry

Tissues were fixed in phosphate buffered formalin (4 %). Immunohistochemical analysis was performed on paraffin-embedded tissue sections using a peroxidase-antiperoxidase system (DAKO, Carpinteria, CA) for the revelation as previously described (Régnier, C.H., et al., J. Biol. Chem. 270 (1995) 25715-25721). Anti-SMAGP antibody was used at 1:1000 dilution.

### Example 7

### SMAGP mRNA expression correlates with metastatic potential in rat and human cancer cell lines

SMAGP mRNA expression was analysed in a panel of isogenic tumor cell lines with distinct metastatic potential. Expression was first evaluated in rat pancreatic, prostatic and mammary tumor cell line systems (Fig.1 A-C). rSMAGP mRNA was either not detectable or only at low levels in the non-metastatic cell lines BSp73-AS, G and MTPa. In contrast, strong expression was found in all metastatic cell lines (BSp73-ASML, AT-1, AT-3, AT-6, MAT-Lu, MAT-LyLu, MTLn2 and MTLn3), except in the poorly metastatic MTC cell line. hSMAGP mRNA Expression in one pancreatic and two colon human isogenic tumor cell systems are shown in Fig. 1 D-F. Overexpression of hSMAGP mRNA was noticed in highly metastatic cells (S2-007, KM12SM, KM12L4, and HCT116-Cl5.5) in comparison to non- or low metastatic cells (S2-028, KM12C and HCT116). Therefore, expression of SMAGP in human and rat cell line systems correlates with their metastatic potential.

### Example 8

### Post-translational modifications of the SMAGP protein

To further characterise the SMAGP protein, polyclonal antibodies were raised against a synthetic peptide corresponding to the last 15 residues of the C-terminal part of hSMAGP, which are conserved to 80% between rat and human SMAGP. In order to demonstrate the translation of the predicted SMAGP protein a Western Blot analysis of protein extracts of rat (MTLn3) and human (HCT116-cl5.5) tumor cells was performed under reducing conditions. A band corresponding to SMAGP of approximately 23 and 25 kDa, respectively (Figure 2 lanes c and g) was observed. This indicated that the size of the expressed proteins was increased about 2-fold than the predicted molecular weight. Since the SMAGP primary sequence contained several putative glycosylation sites, a similar Western Blot analysis of protein extract after deglycosylation with N-glycosidase F (PNGase F), sialidase and O-glycosidase was performed to determine whether SMAGP is post-translationally modified. PNGase F treatment (Figure 2 lanes d and h) had no effect on the apparent molecular weight (MW), indicating that SMAGP is not N-glycosylated.

Sialidase treatment (Figure 1 lanes e and i) led to a small band shift, suggesting that SMAGP is modified by sialic acids. Additional O-glycosidase treatment (Figure 2 lanes f and j) led to a pronounced shift in apparent MW, consistant with the presence of O-glycosylation sites. Finally, treatment with O-glycosidase alone (Figure 2 lane k) had no effect on the MW of SMAGP, confirming the presence of sialic acids attached on O-linked oligosaccharides, which is known to inhibit the action of the O-glycosidase (Fukuda, M., et al., J. Biol. Chem. 262 (1987) 11952-11957). The N-glycosylated transferrin, alpha1-acid and Rnase B glycoproteins were used as positive controls for PNGase F treatment (Figure 2 lanes a and b).

### Example 9

### SMAGP expression and subcellular localisation in human normal tissues

The distribution of SMAGP in a panel of normal human tissues was evaluated by Northern Blot analysis (Fig.3). The expression of SMAGP mRNA is relatively ubiquitous and the highest expression level was detected in placenta. Strong expression was also detected in oesophagus, colon and adipose tissues.

Accordingly, immunohistochemical experiments using the anti-SMAGP antibody on normal colon, breast, lung and biliary tract tissues showed that the SMAGP protein is strongly expressed throughout all organs (Figure 4, A-D). SMAGP expression is restricted to the polarized epithelial structures characterised by cell-cell adhesion leading to cuboïdal cell morphology. In the cells, the protein is mainly localised at the plasma membranes. Moreover, SMAGP is not observed along the whole plasma membrane, but only at lateral membrane domains located at the cell-cell epithelial junctions. The basal and apical membrane sides were devoid of SMAGP. A low staining was also observed in the cell cytoplasm.

### Example 10

### SMAGP expression and subcellular localisation in human colon primary tumors and metastases

SMAGP was examined in primary tumors and metastases that were derived from colon cancer patients. One example of immunohistological staining obtained for tissues coming from the same patient is presented in Fig. 4 (C, normal colon; E and G, primary tumors; F and H, liver metastases). In primary tumors, SMAGP expression can remain quite high (E) or show a weakened expression (G).

Moreover, in some cases the sub-cellular localisation is partially conserved (E) whereas in some others SMAGP was no more recruted to the plasma membrane and became more or less cytoplasmic (G). Similar results were found in the liver metastases (F and H). Thus, for a given patient, various SMAGP phenotypes can be observed in primary tumors and metastases, and even can co-exist inside the same tumors depending on the areas (F). Interestingly, in all primary tumors and metastases studied, strong level of SMAGP protein expression and cell membrane localisation were associated with a conserved epithelial structure (E and F compared to G and H). Similar observations were made in primary tumors and metastases from patients with breast and lung cancers.

### Example 11

### Detection of hSMAGP mRNA expression in human pancreas tissues

hSMAGP mRNA expression was assessed by quantitative RT-PCR (Taqman Technology) in 10 human pancreas carcinomas (Fig. 5, blue bars) and in 5 human normal pancreas tissus (Fig. 5, white bar). The expression of hSMAGP in also measured in the human pancreas tumor cell line Capan I. Overexpression is observed in 5 carcinoma samples, in comparaison to the highest value of expression of hSMAGP reached in normal pancreas tissues, and fold changes are indicated in parenthesis over the corresponding bars.

### Protocol on the Taqman analyses

Reagents of PE were used for carrying out the PCR:
4 µl SybrGreen buffer, 4.8 µl MgCl₂, 3.2 µl dNTDs, 0.2 µl UNG, 0.2 µl Amplitaq Gold, 4 µl Primermix, 1 µl cDNA, 22.6 µl H₂O

The following PCR conditions were applied:
Initial steps: 2 min 50°C, 10 min 95°C, 40 cycles: 15 sec 95°C, 1 min 60°C

Two PCRs (double determination) were carried out to examine the expression of the respective gene for each cDNA. Concurrently, a PCR with xs13 was performed using the same cDNA. The ABI Sequence Detector Program indicates a CT value for each PCR. The CT values of the individual cDNAs were averaged for the respective genes (i.e., mean value of the double determination, for xs13 there exists only one value for each cDNA). The xs13 value for the respective cDNA is then deducted from the above-mentioned averaged values. A mean value is formed only of the differences of the healthy cDNAs. The differences of the chronic pancreatitis, pancreatic sarcinoma and healthy pancreas cDNAs are divided by this mean value. Subsequently, these quotients are raised to the power of base 2 (because during PCR the products are doubled with each reaction). The reciprocals of these values then represent the expression of the respective cDNA compared to healthy cDNA.

### Example 12

### Detection of hSMAGP mRNA expression in human colon tissues

The clinicopathological significance of hSMAGP expression with respect to tumour dissemination was first examined in human tissue samples from colon cancer patients. mRNA expression was assessed by quantitative RT-PCR (Light-Cycler technology) in nine normal colon samples, nine colon primary tumour samples (with known Duke's stadium) and nine liver metastases samples (Fig. 6). Normal and primary tumour samples were derived from the same patient, whereas liver metastasis were not. Strong overexpression was observed in all paired tumours, independently of the Duke's staging, in comparison to normal colon. Moreover, the SMAGP mRNA levels in metastases was higher than the values reached in 6 of 9 primary tumour samples (75% cases). Four normal liver tissue samples were used as control for hSMAGP expression in liver, confirming that the high level of hSMAGP expression in metastases is not resulting from the organ environnement. These results demonstrate overexpression of hSMAGP mRNA in human colon tumours in comparison to normal colon mucosa, and show in 75% of the cases an increased expression in liver metastases versus primary tumours.

Total RNA extracted with RNeasy kit (Qiagen) was subjected to DNase I treatment (Quiagen) before first strand cDNA synthesis with oligo-dT and AMV reverse trancriptase (Roche Diagnostics). Polymerase chain reaction was then performed with a unique enzyme blend containing *Taq* DNA polymerase and *Tgo* DNA polymerase (Roche Diagnostics).

For quantitative RT-PCR, total RNA from frozen colon tissue samples was used. After DNase I treatment, total RNA (1 µg) was reversed transcribed using random hexamers and AMV reverse transcriptase (Roche Diagnostics). Based on the sequence of hSMAGP, two gene-specific primers were designed (H103: 5'-AGA AGA TGG AGC CAG CAC AG-3' (SEQ ID NO:9) and H104: 5'-ATC TGG ACG ATG GCA CTG G-3') (SEQ ID NO:10). These primers are located on two different exons to avoid contamination by genomic DNA. Real-time monitoring of PCR reactions was performed using the LightCycler system and the DNA Master SYBR^{®} Green I Kit (Roche Diagnostics). After optimisation, PCR reaction mixtures contained 4 mM MgCl₂, 0.4 µM of each primer, 2 µl of cDNA solution and 2 µl SYBR Green I in a final volume of 20 µl. Each experiment was performed in duplicate. Calibration curves for both target gene hSMAGP and endogenous reference 18S RNA were generated using serial dilution (1:10, 1:50 and 1:80) of cDNA from the human colon adenocarcinoma cell line KM12SM. For each sample, the relative amounts of target gene and endogenous reference RNAs were determined from the calibration curve. Expression level of hSMAGP mRNA in samples was normalised by dividing the amount of hSMAGP mRNA by the amount of 18S RNA for each sample.

### Example 13

### The impact of SMAGP expression on metastasis formation in the rat

BSp73AS cells, the low metastatic variant of a rat pancreatic adenocarcinoma were stably transfected with SMAGP or the vector alone (mock transfectants). Two mock transfected clones and four clones, that differed slightly in the intensity of SMAGP expression, were selected. These clones as well as untransfected BSp73AS and BSP73ASML cells were injected intraperitoneally or intrafootpad into BDX rats (strain of origin) to control for a potential increase in the metastatic capacity of transfected BSp73AS cells. As the growth behaviour of the two mock transfected and the 4 SMAGP transfected clones did not differ significantly, the data derived with the 2 mock and the 4 SMAGP transfected clones were combined (Table 1).

After intraperitoneal application, BSp73AS and BSp73AS-mock cells grew in large nodules exclusively in the peritoneal cavity, with a mean survival time of BSp73AS and BSp73AS-mock -transplanted rats of 20 days. Rats receiving BSp73ASML cells had a mean survival time of 40 days. The tumor grew in a miliary form in the peritoneal cavity and all rats succumbed with miliary metastasis formation in the lung. From 12 rats receiving SMAGP-transfected BSp73AS cells, 6 developed large nodules in the peritoneal cavity (similar to rats injected with BSp73AS cells), but 6 showed miliary tumor growth in the peritoneal cavity. Four rats had liver metastases and 2 rats each developed metastases in the kidney and the ovary. In 2 rats the diaphragm was heavily infiltrated by the tumor. None of the rats showed lung metastases. These finding demonstrates an increase in the metastatic potential (Table 1A).

To confirm the finding, the tumor cells were injected intrafootpad, where metastatic progression can be more readily followed. When the tumors were not excised after intrafootpad application, all rats developed metastases in the draining popliteal lymph node, but only rats receiving BSp73ASML (5/5) and rats receiving SMAGP-transfected BSp73AS cells (4/5) developed metastases in distant (paraaortic, inguinal and axillary) lymph nodes. In addition, only rats receiving BSp73ASML (5/5) and rats receiving SMAGP-transfected BSp73AS cells (5/5) developed lung metastases. BSp73ASML cells developed miliary metastases, while SMAGP-transfected BSp73AS cells developed large nodules. This finding unequivocally confirms the increased metastatic capacity of SMAGP-expressing tumor cells (Table 1B).

For the estimation of the time required for metastatic settlement, tumor cells were injected intrafootpad and the tumor together with the draining lymph node was excised 28 days thereafter. Data presented in Table 1C refer only to those animals which did not develop a local recurrence due to incomplete removal of the draining lymph node (2 rats with BSp73AS, 4 rats with mock transfected BSp73AS cells and 1 rat with SMAGP transfected BSp73AS cells). From curatively excised rats, 0/5 BSp73ASML-, 3/3 BSp73A-, 6/6 mock-transfected BSp73AS- and 9/14 SMADP-transfected BSp73AS-bearing rats survived. Thus, metastatic settlement proceeds by far more slowly in SMADP-transfected BSp73AS than in BSp73ASML cells, but different to untransfected and mock-transfected BSp73AS cells, in 36% of the rats SMADP transfected BSp73AS cells had migrated beyond the draining lymph node at 28 days after tumor cell application.

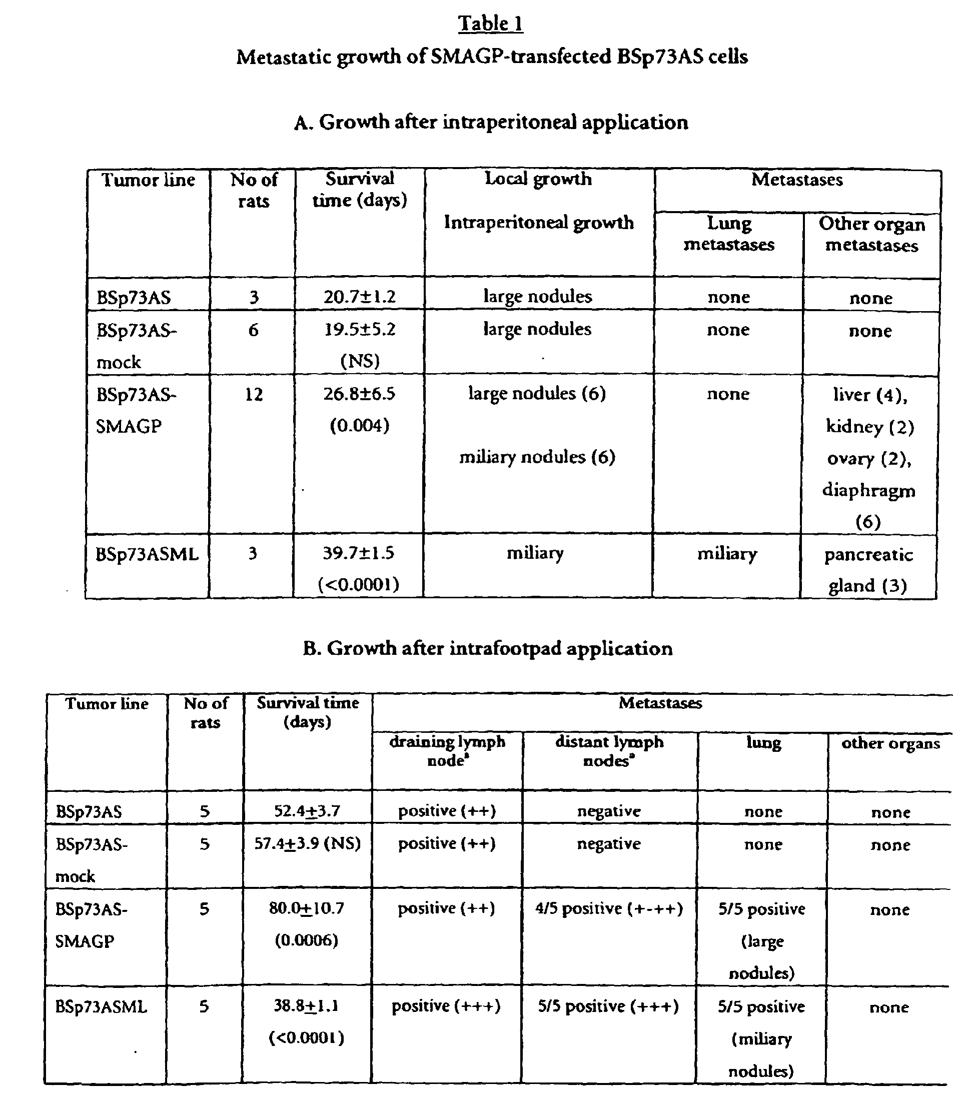

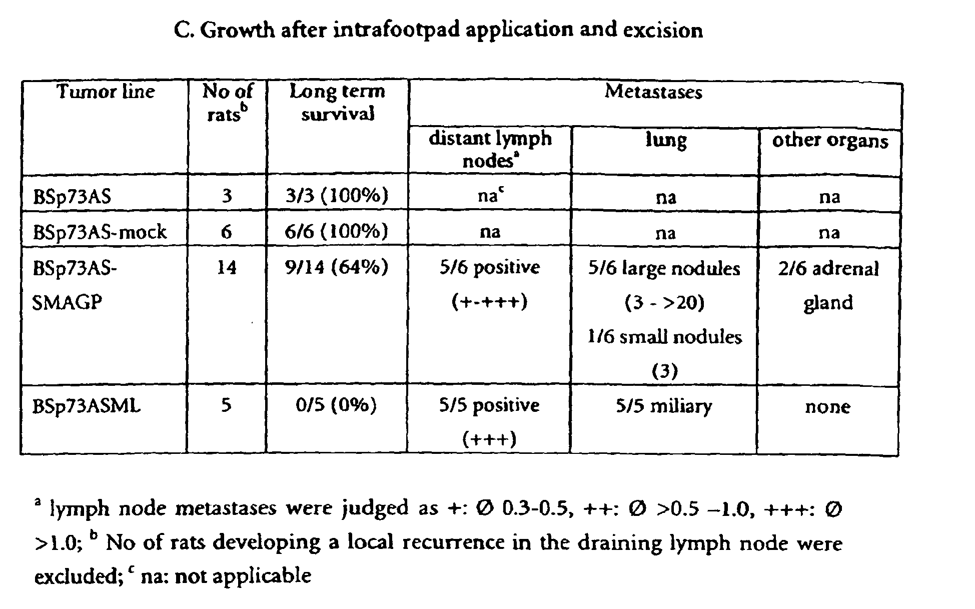

### Example 14

### In vivo metastasis assay

BDX rats were obtained from Jackson Laboratories, Sulzfeld, Germany. Female rats were used for experiments at the age of 8-12 weeks. They received a single injection of 5x10⁵ tumor cells either intraperitoneally (i.p.) or subcutaneously in the hind foot pad (i.f.p.). Where indicated, tumors were excised after i.f.p. application by amputation of the hind foot. In brief, rats were anesthesized with Rompun / Ketanest. The skin was removed by a circular incision with a skalpell at about 0.5 cm below the joint. After sueing the artery and removal of the draining lymph node, the tendons were cut and the hind foot removed by exarticulation. The wound was covered by skin and the skin was sued. Tumor growth was controlled twice per week by either palpation of the abdomen after i.p. application or by measuring the tumor diameter and the diameter of the draining lymph node after i.f.p.application. When the palpable tumor mass reached a mean diameter of 2.5 cm or when rats became cachectic or anemic (that can be estimated by the colour of the eyes and the ears) or short breathing, they were anesthesized and sacrified. Rats were autopsied and all organs were controlled for the presence of metastasis.

### List of References

Alves, F., et al., Pancreas 23 (2001) 227-235
Ausubel I., Frederick M., Current Protocols in Mol. Biol. (1992), John Wiley and Sons, New York
Baines et al., "Purification of Immunoglobulin G (IgG)", In: Methods in Molecular Biology, Vol. 10, pages 79-104, The Humana Press, Inc., 1992
Brattain, M.G., et al., Cancer Res. 41 (1981) 1751-1756
Büttner, R., et al., Mol. Cell. Biol. 11 (1991) 3573-3583
Chambers, A.F., et al., Nat. Rev. Cancer 2 (2002) 563-572
Claas, C., et al., J. Cell. Biol. 141 (1998) 267-280
Coligan et al. (eds.), Current Protocols in Immunology, pp. 9.3.1-9.3.5 and pp. 9.4.1-9.4.11, John Wiley & Sons, 1997
Corti, C., et al., J. Cancer Res. Clin. Oncol. 122 (1996) 154-160
EP-A 0 063 879
EP-A 0 128 018
EP-A 0 173 251
EP-A 0 200 362
Fukuda, M., et al., J. Biol. Chem. 262 (1987) 11952-11957
Gennaro (ed.), Remington's Pharmaceutical Sciences, 19th edition, Mack Publishing Company, 1995
Green, L.L., et al., Nat. Genet. 7 (1994) 13-21
Gunthert, U., et al., Cell 65 (1991) 13-24
Hames, B.D., Higgins, S.G., Nucleic Acid Hybridisation - A Practical Approach (1985) IRL Press, Oxford, England
Hunter, K.W., et al., Cancer Res. 61 (2001) 8866-8872
Isaacs, J.T., et al., Prostate 9 (1986) 261-281
John, C.M., et al., Clin. Cancer Res. 9 (2003) 2374-2383
Lonberg, N., et al., Nature 368 (1994) 856-859
Matzku, S., et al., Invasion Metastasis 3 (1983) 109-123
Mole, "Epitope Mapping", In: Methods in Molecular Biology, Vol. 10, Manson (ed.), pages 105-116, The Humana Press, Inc., 1992
Morikawa, K., et al., Cancer Res. 48 (1988) 6863-6871
Morris (ed.), Epitope Mapping Protocols 25, Humane Press, Inc., 1996
Neri, A., et al., J. Natl. Cancer Inst. 68 (1982) 507-517
Price, "Production and Characterization of Synthetic Peptide-Derived Antibodies", In: Monoclonal Antibodies: Production, Engineering, and Clinical Application, Ritter and Ladyman (eds.), pp. 60-84, Cambridge University Press, 1995
Régnier, C.H., et al., J. Biol. Chem. 270 (1995) 25715-25721
Rosel, M., et al., Oncogene 17 (1998) 1989-2002
Sambrook et al., Molecular Cloning: A Laboratory Manual (1989) Cold Spring Harbor Laboratory Press, New York, USA
Shaper, N.L., et al., J. Mammary Gland Biol. Neoplasia 3 (1998) 315-324
Skubitz, A.P., Cancer Treat. Res. 107 (2002) 305-329
Spieker-Polet, H., et al., Proc. Natl. Acad. Sci. USA 92 (1995) 9348-9352
Steeg, P.S., Nat. Rev. Cancer 3 (2003) 55-63
Taniguchi, S., et al., Clin. Exp. Metastasis 10 (1992) 259-266
Tarbé, N., et al., Anticancer Res. 22 (2002) 2015-2027
Taylor, L.D., et al., Int. Immun. 6 (1994) 579-591
US 2,915,082
Wahl, G.M., et al., Proc. Natl. Acad. Sci. USA 76 (1979) 3683-3687
Welch, D.R., Clin. Exp. Metastasis 15 (1997) 272-306
WO 02/08288
WO 89/06698
Wu, Y.Y., and Rees, J.L., Acta Derm. Venereol. 80 (2000) 2-3
Wurfel, J., et al., Oncogene 18 (1999) 2323-2334

## Claims

1. Method for determining metastasis related occurence of small cell adhesion glycoprotein (SMAGP) in a patient comprising
(i) detecting in a biological sample from a patient suspected to contain tumor cells or a part thereof an amount of nucleic acid encoding SMAGP or an amount of polypeptide SMAGP; and
(ii) comparing the amount of said nucleic acid or polypeptide with a predetermined standard value indicating the decision line for metastasis related SMAGP expression or presence in said cells and therefrom determining metastasis related expression or presence of SMAGP in said patient.

2. Process for determining whether or not a test sample of tissue or fluid of a patient contains tumor cells with metastasis potential or is derived from metastatic tumor cells, wherein the test sample and a second sample originating from non-tumor cells from the same individual or a different individual of the same species are used, which process comprises the following steps:
(a) incubating each respective sample under stringent hybridization conditions with a nucleic acid probe which is selected from the group consisting of:
(i) a nucleic acid sequence of SEQ ID NO: 1, or a fragment thereof;
(ii) a nucleic acid sequence which is complementary to any nucleic acid sequence of (i);
(iii) a nucleic acid sequence which hybridizes under stringent conditions with the sequence of (i); and
(iv) a nucleic acid sequence which hybridizes under stringent conditions with the sequence of (ii); and
(b) determining the approximate amount of hybridization of each respective sample with said probe, and
(c) comparing the approximate amount of hybridization of the test sample to an approximate amount of hybridization of said second sample to identify whether or not the test sample contains a greater amount of the specific nucleic acid or mixture of nucleic acids than does said second sample.

3. Method for the detection of metastasis, comprising
a) incubating a sample of a patient suspected of suffering from metastasis, selected from the group of body fluid, of cells, or of a cell extract or cell culture supernatants of said cells, whereby said sample contains nucleic acids with a nucleic acid probe which is selected from the group consisting of
(i) the nucleic acid shown in SEQ ID NO:1 or a nucleic acid which is complementary to said sequence, and
(ii) nucleic acids which hybridize with one of the nucleic acids from (i) and
b) detecting hybridization, preferably by means of a further binding partner of the nucleic acid of the sample and/or the nucleic acid probe or by X-ray radiography
c) comparing the amount of said nucleic acid hybridization with a predetermined standard value indicating the decision line for metastasis related SMAGP expression in said sample and therefrom determining whether said patient suffers from metastasis.

4. Use of an antibody that binds to the polypeptide SMAGP of the sequence SEQ ID NO:2 in the manufacture of a composition for inhibiting metastasis of tumor cells.

5. Use according to claim 4, wherein the composition is administered to cell cultures in vitro.

6. Use according to claim 4, wherein the composition is a pharmaceutical composition and wherein the pharmaceutical composition is to be administered to a mammalian subject suffering from a tumor.

7. Pharmaceutical composition containing an antibody against SMAGP having the polypeptide sequence SEQ ID NO:2 together with a pharmaceutically acceptable carrier.

## Patentansprüche

1. Verfahren zur Bestimmung von metastasebedingtem Auftreten von kleinem Zelladhäsionsglykoprotein (SMAGP) in einem Patienten umfassend
(i) Nachweis einer Menge an Nukleinsäure, die SMAGP codiert, oder einer Menge von SMAGP Polypeptid in einer biologischen Probe von einem Patienten, die unter Verdacht steht, Tumorzellen oder ein Teil davon zu enthalten; und
(ii) Vergleichen der Menge an Nukleinsäure oder Polypeptid mit einem vorbestimmten Standardwert, der die Entscheidungsgrenze für metastasebedingte SMAGP-Expression oder Anwesenheit in den Zellen angibt und daraus Bestimmung der metastasebedingten Expression oder Anwesenheit von SMAGP im Patienten.

2. Verfahren zur Bestimmung ob eine Testprobe von Gewebe oder von Flüssigkeit eines Patienten Tumorzellen mit Metastasepotenzial enthält oder von metastatischen Tumorzellen stammt, oder nicht, wobei die Testprobe und eine zweite Probe, die von Nicht-Tumorzellen des gleichen Individuums oder eines anderen Individuums der gleichen Spezies stammt, verwendet werden, wobei das Verfahren folgende Schritte umfasst:
(a) Inkubieren der jeweiligen Probe unter stringenten Hybridisierungsbedingungen mit einer Nukleinsäuresonde ausgewählt aus der Gruppe bestehend aus:
(i) einer Nukleinsäuresequenz SEQ ID NO: 1 oder einem Fragment davon;
(ii) einer Nukleinsäuresequenz, die komplementär zu irgendeiner Nukleinsäuresequenz aus (i) ist;
(iii) einer Nukleinsäuresequenz, die unter stringenten Bedingungen mit der Sequenz aus (i) hybridisiert; und
(iv) einer Nukleinsäuresequenz, die unter stringenten Bedingungen mit der Sequenz aus (ii) hybridisiert; und
(b) Bestimmen der ungefähren Menge an Hybridisierung der jeweiligen Probe mit der Sonde, und
(c) Vergleichen der ungefähren Menge der Hybridisierung der Testprobe mit der ungefähren Menge an Hybridisierung der zweiten Probe, um herauszufinden, ob die Testprobe eine größere Menge der spezifischen Nukleinsäure oder einer Mischung von Nukleinsäuren enthält als die zweite Probe oder nicht.

3. Verfahren zum Nachweis von Metastasen, umfassend
a) Inkubieren einer Probe eines Patienten, der unter Verdacht steht, an Metastasen zu leiden, ausgewählt aus der Gruppe von Körperflüssigkeit, von Zellen oder von einem Zellextrakt oder Zellkulturüberständen dieser Zellen, wobei die Probe Nukleinsäuren enthält, mit einer Nukleinsäuresonde, die ausgewählt ist aus der Gruppe bestehend aus
(i) der Nukleinsäure, die in SEQ ID NO:1 gezeigt ist, oder einer Nukleinsäure, die komplementär zu dieser Sequenz ist, und
(ii) Nukleinsäuren, die mit einer der Nukleinsäuren aus (i) hybridisieren, und
b) Nachweis von Hybridisierung, bevorzugt mittels eines weiteren Bindungspartners der Nukleinsäure der Probe und/oder der Nukleinsäuresonde oder durch ein Röntgenradiogramm
c) Vergleichen der Menge an Nukleinsäurehybridisierung mit einem vorbestimmten Standardwert, der die Entscheidungsgrenze für metastasebedingte SMAGP-Expression in der Probe angibt, und daraus Bestimmung, ob der Patient an Metastasen leidet.

4. Verwendung eines Antikörpers, der an das Polypeptid SMAGP der Sequenz SEQ ID NO:2 bindet, zur Herstellung einer Zusammensetzung zur Inhibierung der Metastasenbildung von Tumorzellen.

5. Verwendung nach Anspruch 4, wobei die Zusammensetzung Zellkulturen in vitro verabreicht wird.

6. Verwendung nach Anspruch 4, wobei die Zusammensetzung eine pharmazeutische Zusammensetzung ist und wobei die pharmazeutische Zusammensetzung zur Verabreichung an einen Säuger, der an einem Tumor leidet, vorgesehen ist.

7. Pharmazeutische Zusammensetzung enthaltend einen Antikörper gegen SMAGP mit der Polypeptidsequenz SEQ ID NO:2 zusammen mit einem pharmazeutisch vertretbaren Träger.

## Revendications

1. Procédé de détermination de l'existence de la glycoprotéine d'adhésion de petites cellules (SMAGP) associée aux métastases chez un patient, comprenant:
(i) la détection, dans un échantillon biologique d'un patient suspecté de contenir des cellules tumorales ou une partie de celles-ci, d'une quantité d'acide nucléique codant pour la SMAGP ou d'une quantité de polypeptide SMAGP; et
(ii) la comparaison de la quantité dudit acide nucléique ou polypeptide avec une valeur de référence prédéterminée indiquant la ligne de décision pour l'expression ou la présence de SMAGP associée aux métastases dans lesdites cellules et en conséquence la détermination de l'expression ou de la présence de SMAGP associée aux métastases chez ledit patient.

2. Procédé pour déterminer si un échantillon d'essai de tissu ou de liquide d'un patient contient ou non des cellules tumorales ayant un potentiel de métastase, ou provient de cellules tumorales métastatiques, dans lequel on utilise l'échantillon d'essai et un deuxième échantillon provenant de cellules non tumorales du même individu ou d'un individu différent de la même espèce, ce procédé comprenant les étapes suivantes:
(a) l'incubation de chaque échantillon respectif dans des conditions d'hybridation stringentes avec une sonde d'acide nucléique qui est choisie dans le groupe constitué par:
(i) une séquence d'acide nucléique de SEQ ID N° 1 ou un de ses fragments;
(ii) une séquence d'acide nucléique qui est complémentaire d'une séquence d'acide nucléique quelconque de (i);
(iii) une séquence d'acide nucléique qui s'hybride dans des conditions stringentes avec la séquence de (i); et
(iv) une séquence d'acide nucléique qui s'hybride dans des conditions stringentes avec la séquence de (ii); et
(b) la détermination de la quantité approximative d'hybridation de chaque échantillon respectif avec ladite sonde, et
(c) la comparaison de la quantité approximative d'hybridation de l'échantillon d'essai à la quantité approximative dudit deuxième échantillon pour identifier si l'échantillon d'essai contient ou non une quantité plus grande de l'acide nucléique spécifique ou du mélange d'acides nucléiques que ledit deuxième échantillon.

3. Procédé de détection de métastases, comprenant:
(a) l'incubation d'un échantillon d'un patient suspecté de souffrir de métastases, choisi dans le groupe constitué par un liquide corporel, des cellules ou un extrait de cellules ou des surnageants de culture de cellules desdites cellules, ledit échantillon contenant des acides nucléiques, avec une sonde d'acide nucléique qui est choisie dans le groupe constitué par:
(i) l'acide nucléique représenté par SEQ ID N° 1 ou un acide nucléique qui est complémentaire de ladite séquence; et
(ii) des acides nucléiques qui s'hybrident avec l'un des acides nucléiques de (i) et
(b) la détection d'une hybridation, de préférence à l'aide d'un autre partenaire de liaison de l'acide nucléique de l'échantillon et/ou de la sonde d'acide nucléique ou par radiographie aux rayons X;
(c) la comparaison de la quantité de ladite hybridation d'acide nucléique avec une valeur de référence prédéterminée indiquant la ligne de décision pour l'expression de SMAGP associée aux métastases dans ledit échantillon et en conséquence la détermination du fait que ledit patient souffre ou non de métastases.

4. Utilisation d'un anticorps qui se lie au polypeptide SMAGP de la séquence SEQ ID N° 2 dans la fabrication d'une composition destinée à inhiber la métastase de cellules tumorales.

5. Utilisation selon la revendication 4, dans laquelle la composition est administrée à des cultures de cellules *in vitro.*

6. Utilisation selon la revendication 4, dans laquelle la composition est une composition pharmaceutique et dans laquelle la composition pharmaceutique est destinée à être administrée à un sujet mammifère souffrant d'une tumeur.

7. Composition pharmaceutique contenant un anticorps dirigé contre la SMAGP ayant la séquence polypeptidique SEQ ID N° 2 avec un support pharmaceutiquement acceptable.
